(19) **Europäisches Patentamt / European Patent Office / Office européen des brevets**

(11) **EP 4 715 378 A1**

(12) **EUROPEAN PATENT APPLICATION**
published in accordance with Art. 153(4) EPC

(43) Date of publication:
25.03.2026 Bulletin 2026/13

(21) Application number: 24811014.0

(22) Date of filing: 16.05.2024

(51) International Patent Classification (IPC):
$G01N\ 27/327^{(2006.01)}$ $G01N\ 27/416^{(2006.01)}$

(52) Cooperative Patent Classification (CPC):
G01N 27/327; G01N 27/416

(86) International application number:
PCT/JP2024/018075

(87) International publication number:
WO 2024/242007 (28.11.2024 Gazette 2024/48)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 19.05.2023 JP 2023083361

(71) Applicant: Kao Corporation
Tokyo 103-8210 (JP)

(72) Inventors:
• ASAI, Mitsuo
Chuo-ku, Tokyo 103-8210 (JP)
• ISSHIKI, Nobuyuki
Chuo-ku, Tokyo 103-8210 (JP)

(74) Representative: Hoffmann Eitle
Patent- und Rechtsanwälte PartmbB
Arabellastraße 30
81925 München (DE)

(54) **ENZYME SENSOR**

(57) In one aspect, the present disclosure relates to an enzyme sensor (1) for measuring a concentration of a subject substance in a solution containing the subject substance. The enzyme sensor (1) includes: a working electrode (2) and a standard electrode (3), each of which may be brought into contact with the solution; an enzyme and a redox substance capable of being reversibly oxidized and reduced, the enzyme and the redox substance being each immobilized onto the working electrode (2); and a dV/dt output mechanism (9) capable of outputting, with time, a time differential value dV/dt of a potential difference between the standard electrode (3) and the working electrode (2) caused by changes in concentrations of an oxidized form and a reduced form of the redox substance resulting from a reaction between the subject substance and the enzyme. The potential difference shifts to higher values when the redox substance is oxidized by the reaction between the subject substance and the enzyme, or the potential difference shifts to lower values when the redox substance is reduced by the reaction.

FIG. 1

**Description**

Field of the Invention

The present disclosure relates to a sensor for a specific substance (subject substance) using an enzyme, and a method of measuring a concentration of the specific substance using the sensor.

Background of the Invention

In recent years, along with an increase in health consciousness, there has been a growing need for a sensor that selectively detects an organic substance in a biological fluid, such as sweat, saliva, urine, tears, or blood, for monitoring the state of a body. In particular, non-invasive measurement, which does not damage the body of a subject, such as measurement that does not involve blood collection has been attracting attention. In a biological fluid that can be subjected to non-invasive measurement, the concentration of an organic substance tends to be lower than that in blood, and hence a sensor having higher sensitivity has been required. An enzyme sensor that can selectively detect an organic substance with high sensitivity through utilization of the high selectivity of an enzyme has been considered to be a strong candidate for this type of sensor.

In JP-A-64-88244 (Patent Document 1), a substrate concentration is measured by estimating a steady potential $\Delta V_\infty$, which is generated when a proton produced by an enzyme reaction is captured with a proton-sensitive membrane on a working electrode, from the initial rate of change in potential. JP-A-2019-158650 (Patent Document 2) discloses that an external resistance is disposed between a working electrode and a reference electrode to make the redox reaction of a redox substance reversible. JP-B-2023-553889 (Patent Document 3) discloses a method of measuring a substrate concentration from a change in potential between a working electrode and a reference electrode with time.

Summary of the Invention

In one aspect, the present disclosure relates to an enzyme sensor for measuring a concentration of a subject substance in a solution containing the subject substance, comprising:

a working electrode and a standard electrode, each of which may be brought into contact with the solution;
an enzyme and a redox substance capable of being reversibly oxidized and reduced, the enzyme and the redox substance being each immobilized onto the working electrode; and
a dV/dt output mechanism capable of outputting, with time, a time differential value dV/dt of a potential difference between the standard electrode and the working electrode, which is caused by changes in concentrations of an oxidized form and a reduced form of the redox substance resulting from a reaction between the subject substance and the enzyme,
wherein the potential difference shifts to higher values when the redox substance is oxidized by the reaction between the subject substance and the enzyme, or the potential difference shifts to lower values when the redox substance is reduced by the reaction.
In the present disclosure, the term "concentration of a subject substance" is a concept, which encompasses not only the absolute value of the concentration of the subject substance but also the relative value thereof, and encompasses, for example, the time differential value dV/dt that is in a linear relationship with the absolute value of a substrate concentration that may be used in the calculation of the absolute value of the concentration of the subject substance.

In one aspect, the present disclosure relates to a method for measurement of a subject substance with the enzyme sensor of the present disclosure, comprising:

bringing a solution containing the subject substance into contact with both the working electrode and the standard electrode;
obtaining, with time, a time differential value dV/dt of a potential difference between the standard electrode and the working electrode under a state in which the working electrode and the standard electrode are electrically connected to each other, and a voltage is free from being applied between the working electrode and the standard electrode; and
calculating a concentration of the subject substance through use of the time differential value dV/dt thus obtained and the following linear function (i):

$$c_M = A\frac{dV}{dt} \qquad (i)$$

in the linear function (i), V represents the potential difference between the standard electrode and the working electrode, t represents time, $C_M$ represents the concentration of the subject substance, and A represents a proportionality constant.

In one aspect, the present disclosure relates to another method for measurement of a subject substance with the enzyme sensor of the present disclosure, comprising:

when the electrode system of the enzyme sensor is a two-electrode system, bringing a solution containing the subject substance into contact with the working electrode and the counter electrode (standard electrode), or when the electrode system is a three-electrode system, bringing the solution containing the subject substance into contact with the working electrode, the counter electrode, and the reference electrode (standard electrode) ;
at a time of reaching of a potential of the working electrode to a predetermined potential, which has previously been set, wherein the working electrode and the counter electrode are electrically connected when the electrode system of the enzyme sensor is the two-electrode system, or the working electrode, the counter electrode and the reference electrode are electrically connected when the electrode system of the enzyme sensor is the three-electrode system, applying a predetermined voltage between the counter electrode and the working electrode in each of the two-electrode system and the three-electrode system to flow an electric current between the working electrode and the counter electrode, to return the potential of the working electrode to a value before a start of the measurement;
obtaining, with time, a time differential value dV/dt of a potential difference between the standard electrode and the working electrode; and
calculating a concentration of the subject substance through use of the time differential value dV/dt thus obtained and the following linear function (i):

$$c_M = A\frac{dV}{dt} \qquad\qquad (i)$$

in the linear function (i), V represents the potential difference between the standard electrode and the working electrode, t represents time, $C_M$ represents the concentration of the subject substance, and A represents a proportionality constant.

In one aspect, the present disclosure relates to a measuring system for measuring a concentration of a subject substance in a solution containing the subject substance, the measuring system comprising:

an enzyme sensor;
a signal processing mechanism; and
a display section,
wherein the enzyme sensor includes:

a working electrode and a standard electrode, each of which may be brought into contact with the solution;
an enzyme and a redox substance capable of being reversibly oxidized and reduced, the enzyme and the redox substance being each immobilized onto the working electrode; and
a measuring section capable of, with time, measuring a potential difference between the standard electrode and the working electrode, which is caused by changes in concentrations of an oxidized form and a reduced form of the redox substance resulting from a reaction between the subject substance and the enzyme,

wherein the potential difference shifts to higher values when the redox substance is oxidized by the reaction between the subject substance and the enzyme, or the potential difference shifts to lower values when the redox substance is reduced by the reaction,
wherein the signal processing mechanism calculates the concentration of the subject substance from the potential difference through use of the following linear function (i), and
wherein the display section is capable of displaying the concentration of the subject substance calculated by the signal processing mechanism:

$$c_M = A\frac{dV}{dt} \qquad\qquad (i)$$

in the linear function (i), V represents the potential difference between the standard electrode and the working electrode, t represents time, $C_M$ represents the concentration of the subject substance, and A represents a proportionality constant.

Brief Description of the Drawings

FIG. 1 is a schematic view of an enzyme sensor according to one aspect of the present disclosure.
FIG. 2 is a schematic view of an enzyme sensor according to another aspect of the present disclosure.
FIG. 3 is a schematic view of an enzyme sensor according to another aspect of the present disclosure.
FIG. 4 is a schematic view of an enzyme sensor according to another aspect of the present disclosure.
FIG. 5 is a schematic view of an enzyme sensor according to another aspect of the present disclosure.
FIG. 6 is a schematic view of an enzyme sensor according to another aspect of the present disclosure.
FIG. 7 is a plan view of a working electrode, which is used in each of Example 1 to Example 7 and is formed on a base material.
FIG. 8 is a schematic view for describing a substrate concentration-measuring system used in each of Example 1 and Example 4 to Example 7.
FIG. 9 is a schematic view for describing a substrate concentration-measuring system used in Example 2.
FIG. 10 is a schematic view for describing a substrate concentration-measuring system used in Example 3.
FIG. 11 is a graph showing the time differential value of a working electrode potential that changes with time, the time differential value being obtained by the experiment operation of Example 1.
FIG. 12 is a graph showing a relationship between the time differential value of the working electrode potential obtained in FIG. 11 and a glucose concentration.
FIG. 13 is a graph showing a working electrode potential that changes with time, the potential being obtained by the experiment operation of Example 2.
FIG. 14 is a graph showing a relationship between the time differential value of the working electrode potential obtained by the experiment operation of Example 2 and time.
FIG. 15 is a graph showing the time differential value of a working electrode potential that changes with time, the time differential value being obtained by the experiment operation of Example 3.
FIG. 16 is a graph showing a relationship between the time differential value of the working electrode potential obtained in FIG. 15 and a glucose concentration.

Detailed Description of the Invention

The enzyme sensor disclosed in Patent Document 1 includes the working electrode obtained by immobilizing the enzyme onto the pH-sensitive membrane, and utilizes the fact that the potential of the electrode stabilizes at the steady potential (constant potential) with time. In the enzyme sensor disclosed in Patent Document 1, the following problem is avoided: it takes time for the potential to stabilize at the steady potential, and hence a measuring time becomes longer. Specifically, the measuring time is shortened by calculating the $\Delta V_\infty$ from a specific linear function as an estimated value. However, the method has involved a problem in that a change in concentration of a subject substance in a subject solution with time cannot be measured. In addition, the method has involved the following problem: in detection with an ion-sensitive field effect transistor (ISFET), detection accuracy is low because the proton produced by the enzyme reaction is buffered by the subject solution, and hence a change in potential is liable to be small.

In Patent Document 2, an enzyme sensor that can measure a change in concentration of a subject substance with time is produced as follows: the redox substance capable of being reversibly oxidized and reduced, and an enzyme are immobilized onto its working electrode so that the reaction of the enzyme may be mediated by the redox substance; and the intensity of a signal obtained by amplifying the redox potential of the redox substance with an amplifier such as a field effect transistor is measured. The reaction of the redox substance resulting from the enzyme reaction advances only in one direction, that is, the reaction is the oxidation reaction or the reduction reaction. Hence, there is the following problem of irreversibility: as long as the enzyme reaction continues, the potential of the working electrode continues to rise or fall without stabilizing at any constant potential. In view of the foregoing, in the enzyme sensor, the above-mentioned problem of irreversibility is solved by connecting the working electrode and the reference electrode to each other through the external resistance to accelerate the spontaneous regeneration reaction (oxidation or reduction reaction) of the redox substance serving as an electron transfer mediator. However, the sensor has involved: a problem in that measurement accuracy reduces because the potential of the reference electrode is changed by flowing an electric current through the reference electrode; and a problem in that the reference electrode may be damaged by flowing the electric current through the reference electrode.

In Patent Document 3, before the measurement of the potential between the working electrode and the reference electrode serving as a standard electrode, a potential is applied between the working electrode and the reference electrode for a short time period. Accordingly, a relationship between the change in potential with time and the substrate concentration to be measured becomes complicated, and hence it has been difficult to incorporate a mechanism for determining the substrate concentration into a sensor.

The present disclosure provides, in one aspect thereof, an enzyme sensor that can selectively measure the concentration of a subject substance in a solution containing the subject substance including its change with time with high accuracy.

In one aspect, the enzyme sensor of the present disclosure is an enzyme sensor for measuring the concentration of a subject substance (hereinafter sometimes referred to as "substrate") in a solution containing the subject substance.

The enzyme sensor of the present disclosure includes: a working electrode and a standard electrode, each of which may be brought into contact with the solution; an enzyme and a redox substance that are each immobilized onto the working electrode; and a dV/dt output mechanism electrically connected to the working electrode and the standard electrode.

The dV/dt output mechanism is capable of, with time, outputting the time differential value dV/dt of a potential difference between the standard electrode and the working electrode, which is caused by changes in concentrations of an oxidized form and a reduced form of the redox substance resulting from a reaction between the subject substance and the enzyme. In addition, in the enzyme sensor of the present disclosure, the potential difference shifts to higher values when the redox substance is oxidized by the reaction between the subject substance and the enzyme, or the potential difference shifts to lower values when the redox substance is reduced by the reaction. With such configuration, the enzyme sensor of the present disclosure can selectively measure the concentration of the subject substance including its change with time with high accuracy because of the following reason.

As described above, it has been known that in such a measuring system in which an enzyme is immobilized onto a pH-sensitive membrane as disclosed in Patent Document 1, the potential of its enzyme electrode stabilizes at a constant potential with time.

However, in the measuring system in which the redox substance and the enzyme are immobilized onto the working electrode, the redox substance is oxidized or reduced by the reaction between the substrate and the enzyme (enzyme reaction), but the potential of the working electrode continues to rise or fall without stabilizing at any constant potential. Accordingly, a change in concentration of the substrate with time cannot be monitored. However, when the enzyme and the redox substance are immobilized onto the working electrode, charge generated by the reaction between the substrate and the enzyme can quickly and efficiently transfer to the electrode through the redox substance. Accordingly, even when the concentration of the substrate is low and hence the amount of a product produced by the enzyme reaction is small, a sufficient signal (voltage) intensity is obtained in a short time period in the enzyme sensor. It is conceivable that the change in concentration of the substrate with time can be measured with high accuracy by determining the differential value dV/dt of the signal with respect to time.

As described above, the present disclosure can provide the enzyme sensor that can selectively measure the concentration of the subject substance including its change with time with high accuracy.

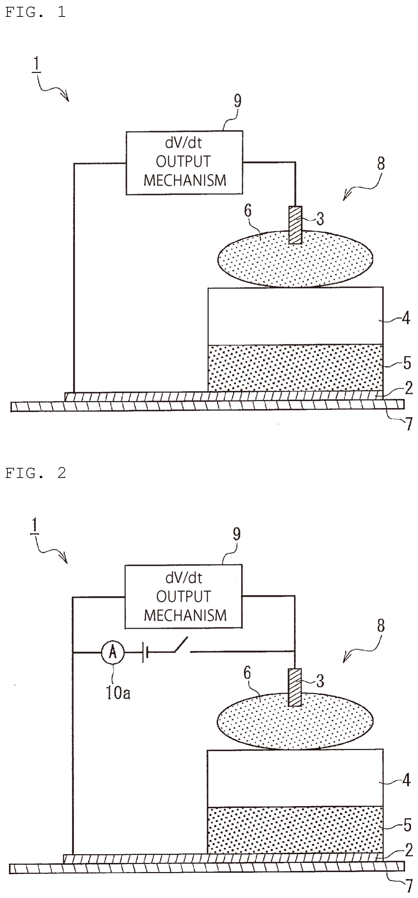

An example of the enzyme sensor of the present disclosure is described below with reference to the drawings. In each of FIG. 1 to FIG. 6, glucose is used as an object subject to measurement (substrate) for convenience of description. However, the sensing object of the enzyme sensor of the present disclosure is not limited thereto.

An enzyme sensor 1 of the present disclosure shown in FIG. 1 is an apparatus that measures the concentration of glucose in a solution 6. The enzyme sensor 1 of the present disclosure includes: a working electrode 2 having immobilized thereonto an enzyme and a redox substance; a sensing part 8 including a counter electrode (standard electrode) 3; and a dV/dt output mechanism 9 electrically connected to the working electrode 2 and the counter electrode 3. The working electrode 2 and the counter electrode 3 may each be brought into contact with the solution 6 containing a subject substance. However, the counter electrode 3 and the working electrode 2 are disposed so as not to be brought into electrical contact with each other. The enzyme is not eluted in the solution 6, and the redox substance is insoluble in the solution 6. The fact that the enzyme is "not eluted" in the solution 6 means that even when the working electrode is brought into contact with the solution 6, the enzyme is substantially free from migrating into the solution because the enzyme is bonded to the working electrode by adsorption or chemical bonding. The fact that the redox substance is "insoluble" in the solution 6 means that the solubility of the redox substance in the solution is small, and hence when the working electrode is brought into contact with the solution, the amount by which the redox substance immobilized onto the working electrode is reduced by its dissolution into the solution is substantially zero. The fact that the amount by which the immobilized redox substance is reduced by its dissolution into the solution is substantially zero means that the solubility of the redox substance in the solution is 100 mg/L or less. In each of FIG. 1 to FIG. 6, reference number 7 represents a base material.

The measurement of the glucose concentration with the enzyme sensor 1 of the present disclosure is performed under a state in which the working electrode 2 and the counter electrode 3 are brought into contact with the solution 6, the working electrode 2 and the counter electrode (standard electrode) 3 are electrically connected to each other, and no voltage is applied between the working electrode 2 and the counter electrode 3, and hence substantially no electric current flows therebetween. Herein, a state in which substantially no electric current flows means a state in which no electric current other than unintended electric current leakage such as a leak current flows. The dV/dt output mechanism 9 outputs, under the state, the change ratio of a potential difference occurring between the counter electrode 3 and the working electrode 2 with respect to time (the "change ratio of the potential difference with respect to time" is hereinafter also referred to as "time differential value dV/dt of the potential difference between the standard electrode and the working electrode" or simply "time differential value dV/dt" in some cases). The "potential difference between the standard electrode and the working electrode" is also referred to as "open circuit voltage (OCV)" or "spontaneous potential." In the present application, the potential difference is sometimes referred to as "working electrode potential."

The "immobilization" of the redox substance and the enzyme onto the working electrode 2 means that the redox substance and the enzyme are each in such a state as not to move with respect to the working electrode 2. It is preferred that the redox substance be directly immobilized onto the working electrode 2. The enzyme may be directly immobilized onto the working electrode 2, or may be indirectly immobilized thereonto through the redox substance. In a preferred aspect, the redox substance forms a redox layer 5 disposed in contact with the working electrode 2, and the enzyme forms an enzyme-immobilized layer 4 disposed in contact with the redox layer. In addition, a layer of the mixture of the enzyme and the redox substance may be formed on the working electrode 2.

Next, an operation in the sensing part 8 is described by taking, for example, a case in which the subject substance (substrate) is glucose as an example.

When the enzyme (glucose oxidase) immobilized onto the working electrode 2 reacts with the glucose in the presence of water and oxygen, the glucose becomes gluconic acid and hydrogen peroxide is produced. Simultaneously, a reduced form out of the redox substance serving as an electron transfer mediator is oxidized into an oxidized form by the hydrogen peroxide. Thus, a ratio between the oxidized form and the reduced form in the electron transfer mediator changes, and the potential difference between the counter electrode 3 and the working electrode 2 changes in accordance with the change.

In the enzyme sensor 1 of the present disclosure, charge generated by the reaction between the substrate and the enzyme can quickly and efficiently transfer to the working electrode 2 through the redox substance because the redox substance and the enzyme are immobilized onto the working electrode 2. Accordingly, even when the concentration of the substrate is low and hence the amount of a product produced by the enzyme reaction is small, a sufficient signal (working electrode potential) intensity is obtained in a short time period in the enzyme sensor 1. A change in concentration of the substrate with time can be measured with high accuracy by determining the time differential value dV/dt of the signal. For example, even when the concentration of the substrate is several tens of $\mu$M (mol/L) or less, the intensity of the signal can be measured. In addition, the enzyme sensor 1 of the present disclosure can selectively perform the measurement of the concentration of the substrate because the sensor utilizes the enzyme reaction.

As described later, in the enzyme sensor of the present disclosure, the time differential value dV/dt of the potential difference between the standard electrode and the working electrode, and the concentration of the substrate are in a linear relationship (first-order correlation), and hence the absolute value of the concentration of the substrate can be easily calculated from measured data on the dV/dt. The enzyme sensor 1 of the present disclosure can monitor the absolute value of the concentration of the substrate with time, because the sensor includes the dV/dt output mechanism 9 capable of outputting the dV/dt with time.

In the enzyme sensor 1 of the present disclosure, the enzyme is not eluted in the solution 6, the redox substance is insoluble in the solution 6, and the enzyme and the substance are each immobilized onto the working electrode 2. Accordingly, the ratio between the oxidized form and the reduced form can be appropriately controlled to a desired ratio by applying a predetermined voltage between the working electrode 2 and the counter electrode 3 to flow an electric current between the working electrode 2 and the counter electrode 3. For example, when the amount of the oxidized form of the redox substance is increased by the enzyme reaction, by flowing the electric current in the direction of reducing the oxidized form, the oxidized form is reduced to decrease the amount of the oxidized form, and thus the ratio between the oxidized form and the reduced form can be returned to that at the time of the start of the measurement. That is, in one aspect, the enzyme sensor 1 of the present disclosure can be repeatedly used. Accordingly, as shown in FIG. 2, in one aspect, the enzyme sensor 1 of the present disclosure preferably further includes a circuit 10a configured to apply a predetermined voltage between the counter electrode 3 and the working electrode 2 as required to enable the flow of an electric current between the working electrode 2 and the counter electrode 3.

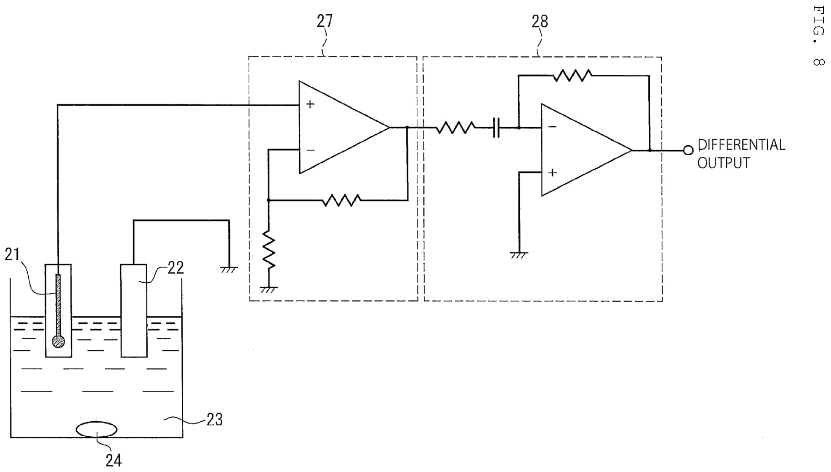

For example, the dV/dt output mechanism 9 capable of outputting the time differential value dV/dt may be a mechanism that can directly output the dV/dt as a signal through use of an electric circuit such as a differentiating circuit (see FIG. 8 to FIG. 10), or the mechanism may include: a measuring device that measures the change of the potential difference occurring between the standard electrode 3 and the working electrode 2, the potential difference being caused by changes in concentrations of the oxidized form and the reduced form of the redox substance resulting from the reaction between the substrate and the enzyme; an A/D converter that subjects the potential difference to A/D conversion; and a central processing unit (CPU) including a computing section that computes the dV/dt from the resultant digital signal. The dV/dt output mechanism 9 may be any mechanism as long as the mechanism can output the time differential value dV/dt.

Next, the fact that the time differential value dV/dt and the absolute value of the concentration of the substrate are in a proportional relationship (linear relationship) is described.

Herein, a case in which the redox substance is oxidized by the reaction between the substrate and the enzyme (enzyme reaction) is mainly described. However, a case in which the redox substance is reduced by the reaction between the substrate and the enzyme (enzyme reaction) is similarly described.

The substrate is oxidized by the enzyme reaction to produce an oxidizing agent such as hydrogen peroxide, and the redox substance in the working electrode is oxidized by the oxidizing agent. At this time, the rate at which the redox substance is oxidized is represented by the following equation on the basis of chemical kinetics.

$$\frac{dc_O}{dt} = -\frac{dc_R}{dt} = kcc_R \tag{1}$$

In the equation (1), $C_O$ represents the oxidized form concentration of the redox substance, $C_R$ represents the reduced form concentration of the redox substance, k represents a reaction rate constant, C represents the concentration of the oxidizing agent, and t represents time. The meanings of those symbols are similarly applicable to the following equations (2) to (9), and the following linear functions (i) and (ii).

When the above-mentioned equation (1) is solved, the concentrations of the oxidized form and reduced form of the redox substance are obtained as represented by the equations (2).

$$\begin{cases} c_O = c_O^0 + c_R^0 \left(1 - e^{-k\int_0^t cdt}\right) \\ c_R = c_R^0 e^{-k\int_0^t cdt} \end{cases} \tag{2}$$

In the equations (2), $C_O^0$ represents the initial oxidized form concentration of the redox substance, and $C_R^0$ represents the initial reduced form concentration of the redox substance. The meanings of those symbols are similarly applicable to the following equations (3) to (9).

The potential difference between the working electrode 2 and the standard electrode 3 is represented by the equation (3) on the basis of the Nernst equation that provides the potential of an electrode having immobilized thereonto a redox substance.

$$V = V_0 + \frac{RT}{nF}\ln\frac{c_O}{c_R} \tag{3}$$

In the equation (3), R represents a gas constant, T represents a temperature (K), F represents Faraday's constant, n represents the number of electrons transferred by the redox reaction of one molecule of the oxidized form, $V_0$ represents the standard redox potential of the redox substance, and V represents the potential of the working electrode 2 that has changed along with changes in concentrations of the oxidized form and reduced form of the redox substance. The meanings of those symbols are similarly applicable to the following equations (4) to (9), and the following linear functions (i) and (ii).

When the equations (2) are substituted into the equation (3), the potential difference between the working electrode 2 and the standard electrode 3 is determined as represented by the equation (4).

$$V = V_0 + \frac{RT}{nF}\ln\frac{c_O^0 + c_R^0 \left(1 - e^{-k\int_0^t cdt}\right)}{c_R^0 e^{-k\int_0^t cdt}} \tag{4}$$

The differentiation of both the sides of the equation (4) with respect to time provides the following equation (5).

$$\frac{dV}{dt} = \frac{RTk}{nF}c + \frac{RT}{nF}\frac{\frac{c_R^0}{c_O^0}kc}{1 + \frac{c_R^0}{c_O^0}\left(1 - e^{-k\int_0^t cdt}\right)} \tag{5}$$

When the initial oxidized form concentration of the redox substance is sufficiently larger than the initial reduced form concentration thereof, the equation (5) can be approximated to the following equation (6) because a second term on its righthand side can be ignored. In the equation (6), the time differential value dV/dt of the potential difference between the working electrode 2 and the standard electrode 3 is in a linear relationship with the oxidizing agent concentration. Further, the oxidizing agent is produced by the enzyme reaction. Accordingly, when the oxidizing agent is in such a steady state that the agent diffuses in the solution without staying on the surface of the working electrode, the oxidizing agent concentration can be regarded as a constant value in accordance with the concentration of the substrate. To be exact, the reaction rate of the enzyme is described by the Michaelis-Menten equation. However, in such a range that the concentration of the subject substance (substrate) is not high, the reaction rate of the enzyme can be regarded as being proportional to the concentration of the subject substance (substrate). Accordingly, the concentration of the oxidizing agent produced by the enzyme reaction can be regarded as being proportional to the concentration of the substrate. Thus, as represented by the equation (7), the time differential value dV/dt of the potential difference between the working electrode 2 and the standard electrode 3 can be regarded as being proportional to the concentration of the substrate. Herein, $c_M$ represents the concentration of the subject substance (substrate).

$$\frac{dV}{dt} = \frac{RT}{nF}kc \qquad (6)$$

$$\frac{dV}{dt} = \frac{RT}{nF}kac_M \qquad (7)$$

In the equation (7), a represents a proportionality constant between the oxidizing agent concentration and the substrate concentration. The meaning of the symbol is similarly applicable to the following equations (8) and (9), and the following linear functions (i) and (ii).

In the foregoing description, it has been hypothesized that the rate of the enzyme reaction is slower than the rate at which the substrate in the solution diffuses. However, even when the rate at which the substrate diffuses is slower than the rate of the enzyme reaction, the rate at which the substrate diffuses is proportional to the concentration of the substrate, and hence the concentration of the oxidizing agent is also proportional to the concentration of the substrate. Accordingly, the following is similarly valid: the change ratio of the potential difference occurring between the counter electrode (standard electrode) 3 and the working electrode 2 with respect to time is proportional to the concentration of the substrate.

It is understood from the foregoing description that the time differential value dV/dt of the potential difference between the working electrode 2 and the standard electrode 3 is proportional to the concentration of the substrate. However, when the concentration of the substrate is calculated from the dV/dt, the concentration may be represented by the equation (8) through use of a proportionality constant A.

$$c_M = A\frac{dV}{dt} \qquad (8)$$

In view of the equation (7), the proportionality constant A of the equation (8) is as represented by the following equation (9). However, the value of the A may be experimentally determined because it is difficult to accurately determine the reaction rate constant k of the redox substance, and the proportionality constant a between the oxidizing agent concentration and the substrate concentration.

$$A = \frac{nF}{RTka} \qquad (9)$$

In the case where the redox substance is reduced by the enzyme reaction as described above, in each of the equations (1) to (9), c represents the concentration of a substance that reduces the redox substance. Accordingly, the reduced form concentration of the redox substance is increased by the enzyme reaction, and hence the potential of the working electrode 2 shifts to lower values. Even in the case where the redox substance is reduced by the enzyme reaction, it is understood from the same discussion as that in the case where the redox substance is oxidized by the enzyme reaction that the change ratio (dV/dt) of the potential difference occurring between the standard electrode 3 and the working electrode 2 with respect to time is proportional to the concentration of the substrate.

In the enzyme sensor of the present disclosure, when the redox substance is oxidized or reduced by the enzyme reaction, the concentration of the substrate, and the time differential value (dV/dt) of the potential difference between the working electrode 2 and the standard electrode 3 are proportional to each other. However, from the viewpoint of high-

8

accuracy measurement, the redox substance is preferably oxidized or reduced by a product produced by the enzyme reaction instead of being directly oxidized or reduced by the enzyme.

When measurement is performed with the enzyme sensor 1 of the present disclosure for a long time period, in, for example, the case where the redox substance is oxidized by the enzyme reaction, the concentration of its reduced form becomes extremely small along with the advance of the enzyme reaction. Under the situation, the influence of slight oxidation of a substance except the redox substance in the working electrode can no longer be ignored, and hence the proportional relationship between the time differential value dV/dt of the potential difference and the concentration of the substrate is gradually impaired. As shown in FIG. 2, the enzyme sensor 1 of the present disclosure preferably includes the circuit 10a as a mechanism that applies a predetermined voltage between the working electrode 2 and the counter electrode (standard electrode) 3 as required to flow an electric current between the working electrode 2 and the counter electrode 3. This is because when the electric current is flowed in the direction of reducing the oxidized form of the redox substance in the circuit 10a, the amount of the oxidized form reduces to increase the amount of the reduced form, and hence the enzyme sensor 1 can be repeatedly used. Meanwhile, when the redox substance is reduced by the enzyme reaction, the electric current has only to be flowed in the direction of oxidizing the reduced form.

In, for example, the case where the redox substance is oxidized as a result of the reaction between the substrate and the enzyme (enzyme reaction), such aspect may be performed by adding the circuit 10a adjusted as follows as a switch: when the potential of the working electrode 2 exceeds a value slightly smaller than a potential for which the following linear function (ii) is valid, a conductive state is established between the working electrode 2 and the counter electrode 3; and for example, when the potential of the working electrode 2 becomes equal to that of the counter electrode 3, a nonconductive state is established therebetween. In the case where the redox substance is reduced as a result of the reaction between the substrate and the enzyme (enzyme reaction), the aspect may be similarly performed by using, as the potential of the working electrode 2, a value slightly larger than the potential for which the following linear function (ii) is valid.

$$\frac{dV}{dt} = \frac{RT}{nF} kac_M \qquad\qquad (ii)$$

The term "slightly smaller value" refers to, for example, a value smaller than the potential for which the linear function (ii) is valid by preferably from 5% to 10%, and the term "slightly larger value" refers to, for example, a value larger than the potential for which the linear function (ii) is valid by more preferably from 5% to 10%.

An electric double layer is present on the surface of the working electrode. Accordingly, in the case where an electric current is flowed to reduce or oxidize the redox substance, when the time period for which the electric current is flowed is excessively short, the above-mentioned electric double layer is merely charged, and hence the redox substance is not oxidized or reduced. Further, charge charged into the electric double layer adversely affects subsequent measurement of the potential difference between the working electrode 2 and the standard electrode 3, and hence the time differential value dV/dt of the potential difference and the concentration of the substrate no longer show any proportional relationship unlike Patent Document 3.

From the viewpoint of sufficiently reducing or oxidizing the redox substance, the time period for which the electric current is flowed is preferably 1 second or more, more preferably 10 seconds or more, still more preferably 20 seconds or more. To reduce the influence of measuring time interruption due to the flow of the electric current, the time period is preferably 300 seconds or less, more preferably 200 seconds or less, still more preferably 100 seconds or less.

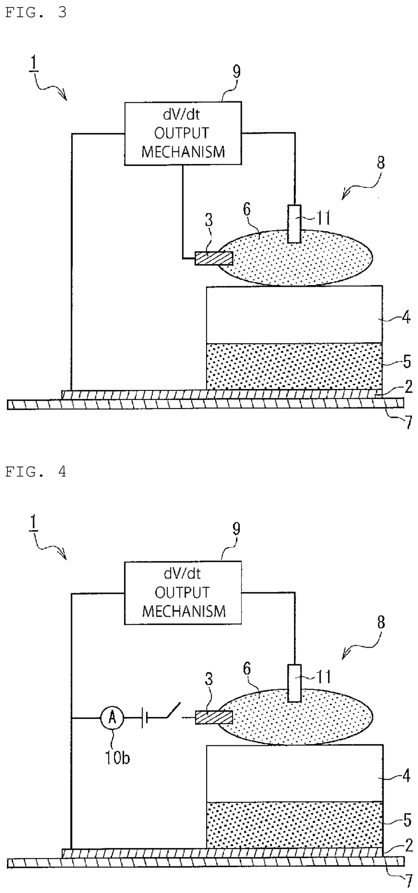

As shown in each of FIG. 1 and FIG. 2, the electrode system of the sensing part 8 may be a two-electrode system formed of the working electrode 2 and the counter electrode (standard electrode) 3. However, as shown in FIG. 3, the electrode system may be a three-electrode system. That is, the electrode system of the enzyme sensor 1 of the present disclosure may be a three-electrode system further including a reference electrode 11, which may be brought into contact with the solution containing the subject substance, independently of the working electrode 2 and the counter electrode 3. In this case, the reference electrode 11 functions as a standard electrode.

As shown in FIG. 4, the enzyme sensor 1 of the present disclosure may include, for example, a circuit 10b as means, which turns its electrode system into the above-mentioned three-electrode system, and can apply a predetermined voltage between the working electrode 2 and the counter electrode 3 as required to flow an electric current therebetween. In this aspect, the redox substance immobilized onto the working electrode 2 can be oxidized or reduced by applying the voltage between the working electrode 2 and the counter electrode 3 as required to flow the electric current through the circuit 10b. Accordingly, for example, when the oxidized form of the redox substance is produced by the reaction between the substrate and the enzyme (enzyme reaction), a change in concentration of the substrate with time can be measured for a longer time period by, for example, shifting the potential of the working electrode 2 to lower values before the start of the measurement to reduce the ratio of the oxidized form at the time of the start of the measurement.

In addition, even when the electrode system is turned into the above-mentioned three-electrode system, the following control is preferably performed because the enzyme sensor 1 can be repeatedly used: a voltage is applied between the working electrode 2 and the counter electrode 3 in, for example, an intermittent manner for a predetermined time period as required to flow an electric current between the working electrode 2 and the counter electrode 3. For example, in the case where the redox substance is oxidized as a result of the reaction between the substrate and the enzyme (enzyme reaction), such aspect may be performed by adding the circuit 10b adjusted, as a switch, as follows: when the potential of the working electrode 2 exceeds a value slightly smaller than a potential for which the linear function (ii) is valid, a conductive state is established between the working electrode 2 and the counter electrode 3; and for example, when the potential of the working electrode 2 becomes equal to the potential of the working electrode 2 at the time of the start of the measurement, a nonconductive state is established therebetween. In the case where the redox substance is reduced as a result of the reaction between the substrate and the enzyme (enzyme reaction), the aspect may be similarly performed by using a value slightly larger than the potential for which the linear function (ii) is valid.

The term "slightly smaller value" refers to, for example, a value smaller than the potential for which the linear function (ii) is valid by preferably from 5% to 10%, and the term "slightly larger value" refers to, for example, a value larger than the potential for which the linear function (ii) is valid by preferably from 5% to 10%.

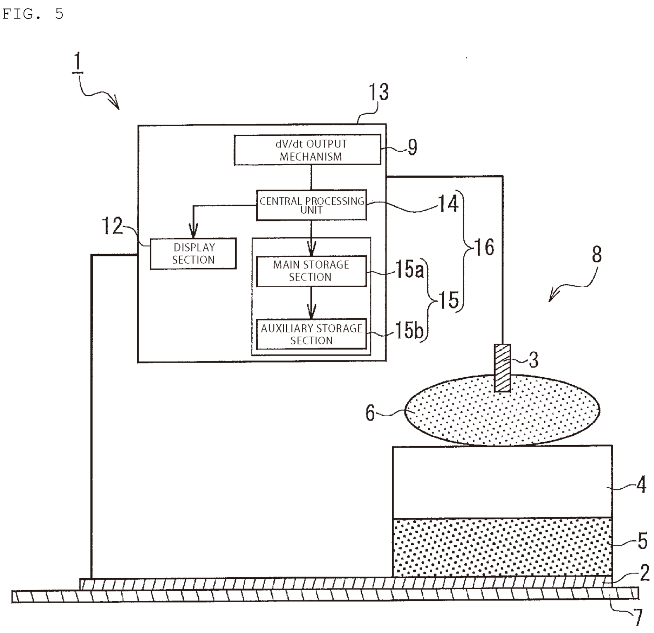

As shown in FIG. 5, the enzyme sensor 1 of the present disclosure may include a computing unit 13 including the dV/dt output mechanism 9, a signal processing mechanism 16, and a display section 12. The signal processing mechanism 16 is preferably a mechanism for calculating, with time, the absolute value of the concentration of the substrate corresponding to the time differential value dV/dt through use of the following linear function (i). However, the mechanism may be a mechanism that displays the relative value of the concentration of the substrate. The display section 12 may be an analog display section or a digital display section.

In one aspect, the signal processing mechanism 16 includes a storage section 15 and a central processing unit 14. The storage section 15 includes a main storage section (memory) 15a and an auxiliary storage section (storage) 15b. The following linear function (i) for calculating the absolute value of the concentration of the substrate from the time differential value dV/dt is stored in the main storage section 15a. In such aspect, a program for calculating the above-mentioned absolute value, the program being stored in the main storage section 15a, is executed by the central processing unit 14 such as a CPU including a computing section and a controlling section to calculate, with time, the absolute value of the concentration of the substrate corresponding to the time differential value dV/dt from the following linear function (i). The absolute value of the concentration of the substrate thus calculated is recorded in the auxiliary storage section 15b. In addition, the absolute value of the concentration of the substrate thus calculated may be subjected to digital processing so as to be capable of being displayed on the display section 12 through control by the controlling section. In another aspect, the signal processing mechanism 16 may be configured as follows: a voltage output by the dV/dt output mechanism 9 is subjected to voltage conversion by, for example, resistance division; and a voltmeter (not shown) is caused to display the resultant value as the absolute value or relative value of the concentration of the substrate calculated from the following linear function (i). In still another aspect, the signal processing mechanism 16 may be an analog configuration in which the voltmeter (not shown) is an analog voltmeter, and the scale of the analog voltmeter is displayed by the concentration of the substrate calculated from the linear function (i).

$$c_M = A\frac{dV}{dt} \qquad\qquad (i)$$

A measuring section is not clearly shown in each of FIG. 1 to FIG. 5. However, in the enzyme sensor 1 of the present disclosure shown in each of FIG. 1 to FIG. 5, the dV/dt output mechanism 9 may include the measuring section.

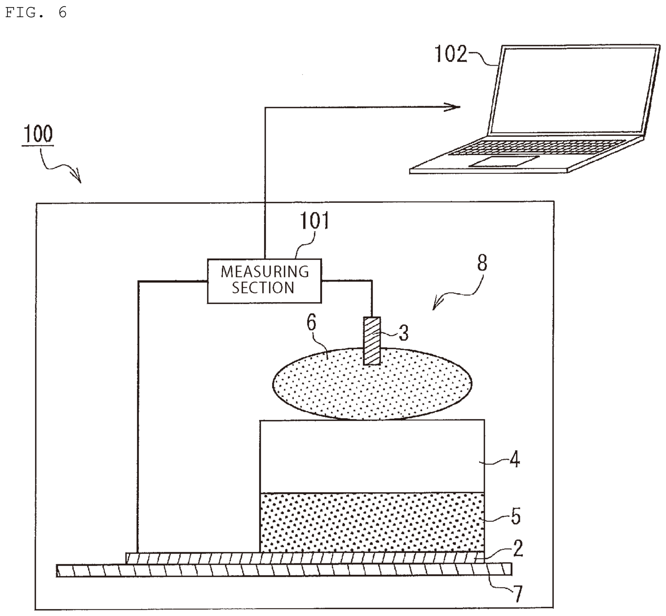

In addition, the enzyme sensor 1 shown in each of FIG. 1 to FIG. 5 includes the dV/dt output mechanism 9. However, as shown in FIG. 6, an enzyme sensor 100 of the present disclosure may be configured to include, instead of the dV/dt output mechanism 9, a measuring section 101 capable of measuring the potential difference (OCV) between the standard electrode and the working electrode 2, with time, and a communication interface for transmitting the resultant digital information on the potential difference to an external information processing terminal 102.

The measuring section 101 is capable of measuring, with time, the potential difference (OCV) between the standard electrode 3 and the working electrode 2 caused by changes in concentrations of the oxidized form and the reduced form of the redox substance resulting from the reaction between the substrate and the enzyme under a state in which the working electrode 2 and the standard electrode 3 are electrically connected to each other, and no voltage is applied between the working electrode 2 and the counter electrode (standard electrode) 3, and hence substantially no electric current is flowed therebetween. The potential difference (OCV) measured by the measuring section 101 shifts to higher values when the redox substance is oxidized by the reaction between the substrate and the enzyme, or the potential difference shifts to lower values when the substance is reduced by the reaction. For example, a potentiostat may be used as the measuring section 101.

The communication interface may be any one of an interface for radio communication and an interface for wire communication. The communication interface means for transferring data from the enzyme sensor of the present disclosure to any other device or system, and examples of the radio communication interface may include technologies, such as wi-fi and Bluetooth (registered trademark).

The information processing terminal 102 is, for example, a digital electronic calculator that can calculate the time differential value dV/dt of the potential difference. For example, the terminal includes: a signal processing mechanism, which includes a central processing unit (CPU) including a computing section and a controlling section, and a storage section including a memory and a storage; an input section; and a display section.

The linear function (i) for converting the time differential value dV/dt into the absolute value of the concentration of the substrate is stored in the memory, and a program for calculating the absolute value of the concentration of the substrate, the program being stored in the memory, is executed by the CPU to output a change in absolute value of the concentration of the substrate corresponding to the time differential value dV/dt with time.

Accordingly, in one aspect, the present disclosure relates to an enzyme sensor for measuring a concentration of a subject substance in a solution containing the subject substance, including:

a working electrode and a standard electrode, each of which may be brought into contact with the solution;
an enzyme and a redox substance capable of being reversibly oxidized and reduced, the enzyme and the redox substance being each immobilized onto the working electrode;
a measuring section capable of measuring, with time, a potential difference between the standard electrode and the working electrode, which is caused by changes in concentrations of an oxidized form and a reduced form of the redox substance resulting from a reaction between the subject substance and the enzyme; and
a communication interface for transmitting the potential difference to an external information processing terminal capable of calculating the time differential value dV/dt of the potential difference,
wherein the potential difference shifts to higher values when the redox substance is oxidized by the reaction between the subject substance and the enzyme, or the potential difference shifts to lower values when the redox substance is reduced by the reaction.

In one aspect, the present disclosure also relates to a measuring system for measuring a concentration of a subject substance in a solution containing the subject substance, the measuring system including:

an enzyme sensor;
a signal processing mechanism; and
a display section,
wherein the enzyme sensor includes:

a working electrode and a standard electrode, each of which may be brought into contact with the solution;
an enzyme and a redox substance capable of being reversibly oxidized and reduced, the enzyme and the redox substance being each immobilized onto the working electrode; and
a measuring section capable of measuring, with time, a potential difference between the standard electrode and the working electrode, which is caused by changes in concentrations of an oxidized form and a reduced form of the redox substance resulting from a reaction between the subject substance and the enzyme,

wherein the potential difference shifts to higher values when the redox substance is oxidized by the reaction between the subject substance and the enzyme, or the potential difference shifts to lower values when the redox substance is reduced by the reaction,
wherein the signal processing mechanism calculates the concentration of the subject substance from the potential difference through use of the following linear function (i), and
wherein the display section is capable of displaying the concentration of the subject substance calculated by the signal processing mechanism:

$$c_M = A\frac{dV}{dt} \qquad (i)$$

in the linear function (i), V represents the potential difference between the standard electrode and the working electrode, t represents time, $C_M$ represents the concentration of the subject substance, and A represents a proportionality constant.

No matter what method the dV/dt output mechanism 9 and the measuring section 101 are each formed by, the mechanism and the section each have a definite input impedance as long as the mechanism and the section each measure a potential difference. Accordingly, a minute current (leak current) flows through the working electrode 2 connected to the dV/dt output mechanism 9 or the measuring section 101 owing to a load effect exhibited by the mechanism or the section. When the redox substance is oxidized by the reaction between the substrate and the enzyme (enzyme reaction), the oxidized form of the redox substance is reduced by the minute current, and when the redox substance is reduced by the reaction between the substrate and the enzyme (enzyme reaction), the reduced form thereof is oxidized by the minute current. When the reduction of the oxidized form or the oxidation of the reduced form by the minute current can no longer be ignored as compared to the amount of the oxidized form or the reduced form produced by the reaction between the substrate and the enzyme (enzyme reaction), the equation (1) (chemical kinetic equation) and the linear function (i) become no longer valid for the potential difference between the working electrode 2 and the standard electrode 3 to be measured.

In order that the reduction of the oxidized form or the oxidation of the reduced form by the minute current described above may be substantially free from affecting the enzyme sensor, the minute current flowing through the working electrode 2 needs to be made sufficiently small by making the input impedance of the dV/dt output mechanism 9 or the measuring section 101 sufficiently high in accordance with an ordinary method. For example, the use of an operational amplifier can easily set the input impedance of the dV/dt output mechanism 9 or the measuring section 101 to such a large value that the load effect thereof can be ignored. For example, the input impedance is set so that the amount of the redox substance reduced or oxidized by the electric current generated by the load effect may be preferably 50% or less, more preferably 10% or less of the redox substance oxidized or reduced by the enzyme reaction.

Whether or not the input impedance of the dV/dt output mechanism 9 or the measuring section 101 is sufficiently high can be recognized by the establishment of the following situation: the potential of the working electrode during the measurement shifts to higher values when the redox substance is oxidized as a result of the reaction between the substrate and the enzyme; or the potential shifts to lower values when the redox substance is reduced as a result of the reaction between the substrate and the enzyme.

The respective configurations of the enzyme sensor of the present disclosure are exemplified in more detail below.

<Sensing Part>

[Redox Substance]

The redox substance for forming the enzyme sensor 1 of the present disclosure is not particularly limited as long as the redox substance can transfer an electron to an electrode, and a conventionally known substance may be used. Examples of the redox substance may include: Prussian blue; Meldola blue; tetrathiafulvalene; quinones, such as hydroquinone and 1,4-naphthoquinone; ferrocene; a ferrocene derivative; potassium ferrocyanide; a ferricyanide; an osmium complex; and p-aminophenol.

The redox layer 5 may be formed by: dropping or applying a solution containing the redox substance onto the working electrode 2; and then drying the solution. The solution is, for example, a dispersion obtained by dispersing the redox substance in a dispersion medium. The redox substance that is not immobilized is preferably removed by washing, and for example, pure water or a buffer solution may be used as a washing liquid. The total amount of the redox substance to be immobilized onto the working electrode 2 is appropriately determined in accordance with, for example, the applications (the kind of the substrate) of the enzyme sensor 1 of the present disclosure, for example, so that the linear function (ii) may be valid during a desired measuring time. In addition, the solution is preferably mixed with a conductive material because the conductivity of the redox layer 5 is improved. Examples of the conductive material to be incorporated into the redox layer 5 may include conductive carbon materials. Of those, a carbon nanotube (CNT) is preferred because of the following reason: the CNT has satisfactory conductivity and a large specific surface area, and hence enables satisfactory performance of the immobilization of the enzyme and the redox substance onto the working electrode 2. The carbon nanotube may be any one of a single-walled carbon nanotube and a multiwalled carbon nanotube.

[Reducing Agent]

When the redox substance is oxidized by the enzyme reaction, it is preferable that a reducing agent is previously incorporated into the redox substance layer 5 in addition to the redox substance. When the reducing agent is previously incorporated into the redox substance layer, the potential of the working electrode at the time of the start of the measurement can be set to even lower values, and hence the time differential value dV/dt of the potential for which the linear function (i) is valid can be measured for a longer time period.

The kind of the reducing agent is not particularly limited as long as the agent can reduce the redox substance, and a conventionally known agent may be used. Examples of the reducing agent may include: thiol compounds, such as mercaptopropanediol, mercaptoethanol, mercaptomethanol, and thiophenol; thiourea; ascorbic acid; oxalic acid; formic acid; gallic acid; uric acid; lithium aluminum hydride; sodium borohydride; and hydrazine.

From the viewpoint that the time differential value dV/dt of the potential for which the linear function (i) is valid can be measured for a long time period, a molar ratio (reducing agent/redox substance) between the reducing agent and the redox substance in the redox substance layer is preferably 0.1 or more, more preferably 0.5 or more, still more preferably 0.8 or more. From the viewpoint of making the oxidized form concentration of the redox substance larger than the reduced form concentration thereof to maintain a proportional relationship between the time differential value dV/dt of the potential difference and the concentration of the substrate, the ratio is preferably 30 or less, more preferably 20 or less, still more preferably 10 or less.

[Oxidizing Agent]

When the redox substance is reduced by the enzyme reaction, it is preferable that an oxidizing agent is previously incorporated into the redox substance layer 5 in addition to the redox substance. When the oxidizing agent is previously incorporated into the redox substance layer, the potential of the working electrode at the time of the start of the measurement can be set to even higher values, and hence the time differential value dV/dt of the potential for which the linear function (i) is valid can be measured for a longer time period.

The kind of the oxidizing agent is not particularly limited as long as the agent can reduce the redox substance, and a conventionally known agent may be used. Examples of the oxidizing agent may include: peroxides such as hydrogen peroxide; potassium nitrate; and chromic acid.

From the viewpoint that the time differential value dV/dt of the potential for which the linear function (i) is valid can be measured for a long time period, a molar ratio (oxidizing agent/redox substance) between the oxidizing agent and the redox substance in the redox substance layer is preferably 0.1 or more, more preferably 0.5 or more, still more preferably 0.8 or more. From the viewpoint of making the reduced form concentration of the redox substance larger than the oxidized form concentration thereof to maintain a proportional relationship between the time differential value dV/dt of the potential difference and the concentration of the substrate, the ratio is preferably 30 or less, more preferably 20 or less, still more preferably 10 or less.

[Enzyme]

The enzyme for forming the sensing part 8 is not particularly limited as long as the enzyme can exchange an electron through its reaction, and any enzyme is appropriately applied in accordance with the sensing object (substrate). However, an enzyme that selectively reacts with the substrate is preferred. For example, when glucose in blood, urine, or the like is the sensing object, examples of the enzyme may include glucose oxidase (GOD) and glucose dehydrogenase (GDH). In addition to the foregoing, an enzyme that may be used in accordance with the sensing object in a biological fluid is, for example, lactate oxidase, lactate dehydrogenase, urease, uricase, an amino acid oxidase, bilirubin oxidase, cholesterol oxidase, an alcohol oxidase, or an alcohol dehydrogenase.

The enzyme-immobilized layer 4 may be formed by, for example, dropping or applying a solution containing the enzyme and a binder such as chitosan onto the redox layer 5, and then drying the solution. The above-mentioned solution is, for example, the mixed liquid of a solution having dissolved thereinto the enzyme and a solution having dissolved thereinto the binder. The enzyme that is not immobilized is preferably removed by washing, and for example, pure water or a buffer solution may be used as a washing liquid. In addition to chitosan, polyvinyl butyral, cellulose acetate, polyvinyl alcohol, poly-L-lysine, a 2-methacryloyloxyethyl phosphocholine polymer, fibroin, or the like may be used as the binder. In addition, the enzyme-immobilized layer may be formed by crosslinking the molecules of the enzyme through a covalent bond through use of a crosslinking agent such as glutaraldehyde.

Thus, an enzyme-modified electrode in which the enzyme and the redox substance are immobilized onto the working electrode 2 is obtained. The total amount of the redox substance to be immobilized onto the working electrode 2 and the total amount of the enzyme to be immobilized thereonto are appropriately determined in accordance with, for example, the applications (the kind of the substrate) of the enzyme sensor of the present disclosure, for example, so that the linear function (ii) may be valid during a desired measuring time.

[Working Electrode, Reference Electrode, and Counter Electrode]

The working electrode 2, the counter electrode 3, and the reference electrode 11, which is incorporated as required, for forming the enzyme sensor 1 of the present disclosure are each formed of, for example, a conductive layer formed on the base material 7. A material for each of those conductive layers is selected in consideration of a product of a reaction with the enzyme. Typical examples of the material may include: a metal, such as gold, palladium, platinum, rhodium, indium, or iridium; and a carbon material. Of those, gold or platinum is preferred. However, a conductive material that does not react with the solution containing the subject substance is permitted. It is preferred that the counter electrode 3 be a platinum electrode, the working electrode 2 be a gold electrode, and the reference electrode 11 be an electrode obtained by forming a Ag/AgCl electrode on gold. The conductive layer is formed by, for example, depositing such metal from the vapor onto an arbitrary position on the base material 7. Alternatively, the various electrodes may each be obtained by bonding a separately prepared metal thin film to the base material 7. In addition, the conductive layers (electrodes) may each be formed by a conventionally known method, such as vacuum deposition, an electron beam, sputtering, plating, CVD, ion plating coating, or inkjet printing, in accordance with the material therefor. The counter electrode 3 may be formed by using a metal wire or a metal plate without use of the base material and the conductive layer. The reference electrode may be formed by sealing a standard electrode substance such as Ag/AgCl in a glass tube without use of the base material and the conductive layer. Further, in a two-electrode system that includes the counter electrode 3 but is free of the reference electrode 11, an electrode serving as a standard electrode such as a Ag/AgCl electrode may be used as the counter electrode 3 for clarifying the standard of the potential of the working electrode 2.

[Base Material]

A material for the above-mentioned base material may be a sheet including, for example, a polyimide (PI) resin, a polyester resin, a polyamide resin, an epoxy resin, or a polysulfone resin, or may be a film-shaped flexible material formed of such resin.

[Method of measuring Concentration of Subject substance (Substrate)]

Next, a method of measuring the concentration of a subject substance (sensing object) of the present disclosure (hereinafter sometimes referred to as "measurement method of the present disclosure") with the enzyme sensor of the present disclosure is described.

The measurement method of the present disclosure is a method of measuring the concentration of the subject substance, with time (e.g., a biological substance), and in one aspect, includes at least the following steps (A) and (B).

The measurement method of the present disclosure may further include a step (C) in addition to the steps (A) and (B).

In one aspect, in the measurement method of the present disclosure, the following steps (A) to (C) advance in a substantially simultaneous manner because a time differential value dV/dt is obtained with time:

(A) bringing the solution 6 containing the subject substance into contact with both the working electrode and the standard electrode;

(B) obtaining, with time, the time differential value dV/dt of a potential difference between the standard electrode and the working electrode under a state in which the working electrode and the standard electrode are electrically connected to each other, and no voltage is applied between the working electrode and the standard electrode, and hence substantially no electric current flows therebetween; and

(C) obtaining the absolute value of the concentration of the substrate corresponding to the time differential value dV/dt from the following linear function (i) through use of the time differential value dV/dt.

$$c_M = A\frac{dV}{dt} \qquad\qquad (i)$$

The time differential values dV/dt obtained in the above-mentioned step (B) are each converted into the absolute value of the concentration of the substrate in the above-mentioned step (C). Accordingly, the measurement method of the present disclosure including the above-mentioned steps (A) to (C) enables the measurement of a change in absolute value of the concentration of the substrate with time.

When only a relative change in concentration of the substrate is to be measured, the time differential value dV/dt obtained in the above-mentioned step (B) has only to be used as it is without performance of the above-mentioned step (C).

The above-mentioned step (C) may be performed with the enzyme sensor of the present disclosure, or may be performed with an external information processing terminal. When the above-mentioned step (C) is performed with the external information processing terminal, the above-mentioned step (B) includes transmitting the time differential value dV/dt to the external information processing terminal after obtaining the time differential value dV/dt.

In the measurement method of the present disclosure, the following step (D) may be further performed after the above-mentioned steps (A) to (C) have been performed for a predetermined time period:

(D) at the time of the reaching of the potential of the working electrode to a predetermined potential, which has previously been set, applying a predetermined voltage between the working electrode and the counter electrode to flow an electric current between the working electrode and the counter electrode, to return the potential of the working electrode to a value before the start of the measurement.

The above-mentioned step (D) may be performed in each of the following cases: a case in which the electrode system of the enzyme sensor is a two-electrode system formed of the working electrode and the counter electrode; and a case in which the system is a three-electrode system formed of the working electrode, the counter electrode, and the reference electrode. The above-mentioned step (D) may be performed when the enzyme sensor includes, as a switch, the circuit 10a or 10b adjusted so that a conductive state or a nonconductive state may be established between the working electrode and the counter electrode in accordance with the potential of the working electrode.

As described above, when the amount of a reduced form or the amount of an oxidized form in a measuring system becomes excessively small as a result of the enzyme reaction, a correlation between the time differential value dV/dt and the concentration of the substrate deteriorates. The time period for which the concentration of the subject substance is continuously measured with the enzyme sensor of the present disclosure is, for example, the maximum time period for which the linear function (i) is valid.

When the redox substance is oxidized by the enzyme reaction between the substrate and the enzyme, the term "predetermined potential, which has previously been set" refers to a value slightly smaller than the potential of the working electrode 2 for which the linear function (i) is valid. When the redox substance is reduced by the enzyme reaction between the substrate and the enzyme, the term refers to a value slightly larger than the potential of the working electrode 2 for which the linear function (i) is valid.

The term "slightly smaller value" refers to, for example, a value smaller than the potential of the working electrode 2 for which the linear function (i) is valid by preferably from 5% to 10%, and the term "slightly larger value" refers to, for example, a value larger than the potential of the working electrode 2 for which the linear function (i) is valid by preferably from 5% to 10%.

In one aspect, in the enzyme sensor of the present disclosure to be used in the measurement method of the present disclosure, the standard electrode is the reference electrode for forming the three-electrode system. That is, in this aspect, the enzyme sensor includes the working electrode, the counter electrode, and the reference electrode (standard electrode). A ratio between the oxidized form and reduced form of the redox substance can be set to a desired value before the start of the measurement by adjusting the voltage to be applied between the working electrode and the counter electrode so that the potential of the working electrode with respect to the reference electrode (standard electrode) may be a potential corresponding to the desired ratio between the oxidized form and the reduced form. Thus, for example, when the oxidized form of the redox substance is produced by the reaction between the substrate and the enzyme (enzyme reaction), a change in concentration of the substrate with time can be measured for a longer time period by, for example, shifting the potential of the working electrode to lower values before the start of the measurement to reduce the ratio of the oxidized form at the time of the start of the measurement.

The applications of the enzyme sensor of the present disclosure are not particularly limited as long as the property of a redox enzyme called substrate specificity can be utilized in the applications. Examples thereof may include: a biomedical sensor whose sensing object is an organic substance or a body fluid in a biological sample; and a biosensor that senses a virus, an antibody, or the like in an environmental sample. More specific examples thereof may include: a blood glucose level sensor that measures the concentration of glucose in blood; a urine glucose level sensor that measures the concentration of glucose in urine; a lactic acid sensor that measures the concentration of lactic acid in sweat; and a sensor for a virus present in food, sewage, or the like.

Examples

The present disclosure is described in more detail below by way of Examples. However, Examples are exemplary and the present disclosure is not limited to these Examples.

(Example 1)

[Working Electrode]

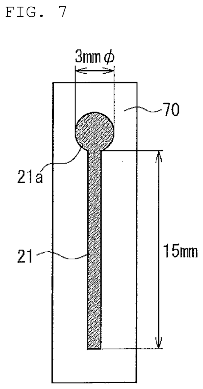

Au was deposited from the vapor in a vacuum onto a base material (polyimide film, thickness: 125 $\mu$m) 70 through a metal mask so as to have a thickness of 50 nm. Thus, a working electrode 21 having a pattern shown in FIG. 7 was formed. Next, 5 $\mu$l of a mixed solution obtained by mixing a 0.2 wt% aqueous dispersion of carbon nanotubes (manufactured by TUBALL) and an aqueous solution containing 1 wt% Prussian blue (redox substance, manufactured by Sigma-Aldrich Co. LLC) at a volume ratio of 10:1 was dropped onto a circular section 21a having a diameter of 3 mm out of the working electrode 21, and was dried in a thermostat at 80°C for 10 minutes. Thus, a Prussian blue-containing carbon nanotube layer was formed as a redox layer.

Next, 10 $\mu$l of a mixed solution obtained by mixing phosphate-buffered saline (PBS solution, pH=7.2, manufactured by Life Technologies) containing 1 wt% glucose oxidase (enzyme, manufactured by Sigma-Aldrich Co. LLC) and a 2 wt% acetic acid solution (manufactured by FUJIFILM Wako Pure Chemical Corporation) containing 1 wt% chitosan (manufactured by Sigma-Aldrich Co. LLC) at a volume ratio of 1:2 was dropped onto the Prussian blue-containing carbon nanotube layer (redox layer), and was dried at 25°C for 20 hours. After that, the enzyme that was not immobilized was washed off with the PBS solution, and the residue was dried at normal temperature to form an enzyme-immobilized layer. Thus, an enzyme-modified electrode in which the enzyme and the redox substance were immobilized onto the working electrode 21 was obtained.

When the produced working electrode is immersed in a glucose solution, the redox substance (Prussian blue) is oxidized by hydrogen peroxide produced by an enzyme reaction.

[Counter Electrode (Standard Electrode)]

A silver/silver chloride electrode was used as a counter electrode.

[Method of measuring Concentration of Substrate]

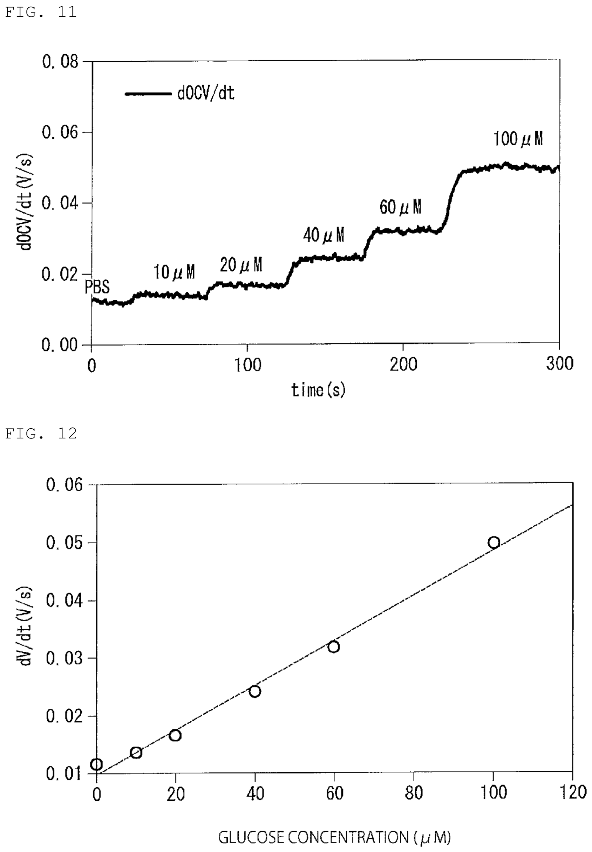

The concentration of a subject substance (substrate) was measured with the measuring system of FIG. 8 as described below. As shown in FIG. 8, the enzyme-modified electrode in which the enzyme and the redox substance were immobilized onto the working electrode 21 was connected to the non-inverting input terminal of a non-inverting amplifier circuit 27. A counter electrode 22 was connected so as to have a ground potential. The enzyme-modified electrode and the counter electrode 22 were immersed in 10 mL of a PBS solution (subject solution), and the subject solution was stirred with a stirring bar 24 at 500 rpm. First of all, the measurement of the time differential value dV/dt of the potential of the working electrode 21 output by a differentiating circuit 28 was started. 20 seconds after that, 1 μL of a 100 mM D-glucose solution was added to the PBS solution, which was being stirred, to adjust the concentration of glucose in the subject solution to 10 μM. After that, the glucose concentration was continuously changed to 20 μM, 40 μM, 60 μM, and 100 μM. As a result, there was obtained such a graph in which the time differential value dV/dt of the potential of the working electrode 21 for each glucose concentration was continuously measured as shown in FIG. 11.

[Determination of Amount of Subject substance]

Plots (calibration curve data) in which the glucose concentration and the time differential value dV/dt were in a proportional relationship as shown in FIG. 12 were obtained from the time differential value dV/dt for each glucose concentration shown in FIG. 11. The plots were consistent with the following linear function (i). Then, the following linear function (i) was stored in the memory of the above-mentioned external information processing terminal (digital electronic calculator), and a program for calculating the absolute value of the concentration of the substrate through use of the linear function (i), the program being similarly stored in the memory, was executed. Thus, the glucose concentration corresponding to the time differential value dV/dt was able to be output.

$$c_M = A \frac{dV}{dt} \qquad (i)$$

(Example 2)

[Working Electrode]

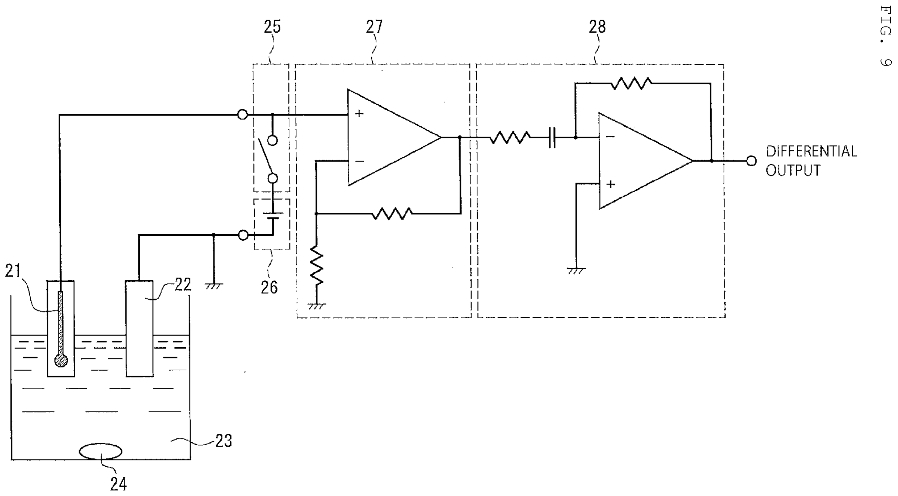

The working electrode 21 (see FIG. 9) and an enzyme-modified electrode including the electrode were each produced in the same manner as in Example 1.

[Counter Electrode (Standard Electrode)]

A copper electrode was used as the counter electrode 22.

[Method of measuring Concentration of Substrate]

The concentration of a subject substance (substrate) was measured with the measuring system of FIG. 9 as described below.
As shown in FIG. 9, the enzyme-modified electrode in which the enzyme and the redox substance were immobilized onto the working electrode 21, and the counter electrode 22 were connected to each other through a switch 25 controlling energization and a power supply 26 providing a predetermined potential, and then the enzyme-modified electrode was connected to the non-inverting input terminal of the non-inverting amplifier circuit 27. The enzyme-modified electrode and the counter electrode 22 were immersed in 10 mL of a PBS solution of 30 $\mu$M glucose (subject solution), and the subject solution was stirred with the stirring bar 24 at 500 rpm. After that, the following (1) to (3) were performed.

(1) A change in potential of the working electrode 21 was measured with the non-inverting amplifier circuit 27 under a state in which the switch 25 was opened so that the working electrode 21 and the counter electrode 22 were not energized. Thus, the following result was obtained: the potential increased to higher values with time.

(2) When the potential of the working electrode 21 measured with the non-inverting amplifier circuit 27 reached 0.180 V, the switch 25 was closed, and a voltage of 0.115 V was applied between the working electrode 21 and the counter electrode 22 for 10 seconds to energize the working electrode 21 and the counter electrode 22. Thus, the potential of the working electrode 21 was returned to 0.115 V.

(3) Immediately after the potential of the working electrode 21 had returned to the predetermined value (0.115 V), the switch 25 was opened so that the working electrode 21 and the counter electrode 22 were not energized. After that, a change in potential of the working electrode 21 was measured with the non-inverting amplifier circuit 27 again.

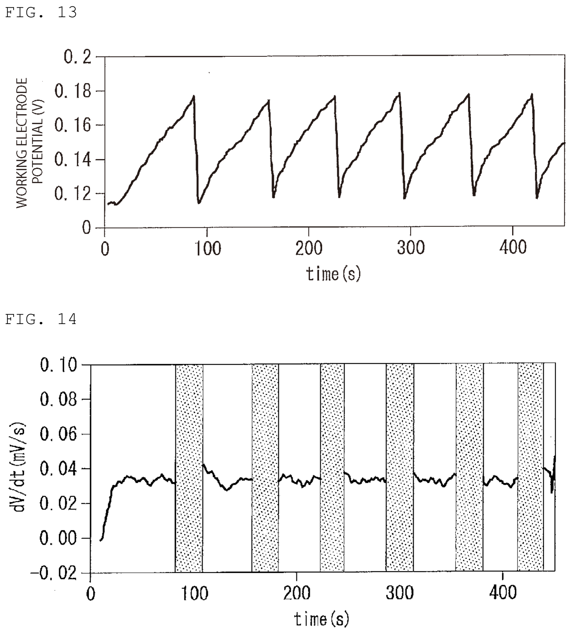

After that, the above-mentioned (2) and (3) were each repeated six times to provide a graph showing the potential of the working electrode 21 that changed with time as shown in FIG. 13. Further, the time differential value of the potential of the working electrode 21 was measured with the differentiating circuit 28 connected to the non-inverting amplifier circuit 27. Thus, there was obtained a graph shown in FIG. 14 showing a constant time differential value dV/dt with respect to the potential of the working electrode 21 that changed with time. While energization is established between the working electrode 21 and the counter electrode 22, in FIG. 14, the measurement of the time differential value dV/dt of the potential of the working electrode 21 is stopped.

The input impedance of the non-inverting input terminal of the non-inverting amplifier circuit 27 was 1 M$\Omega$ or more.

(Example 3)

[Working Electrode]

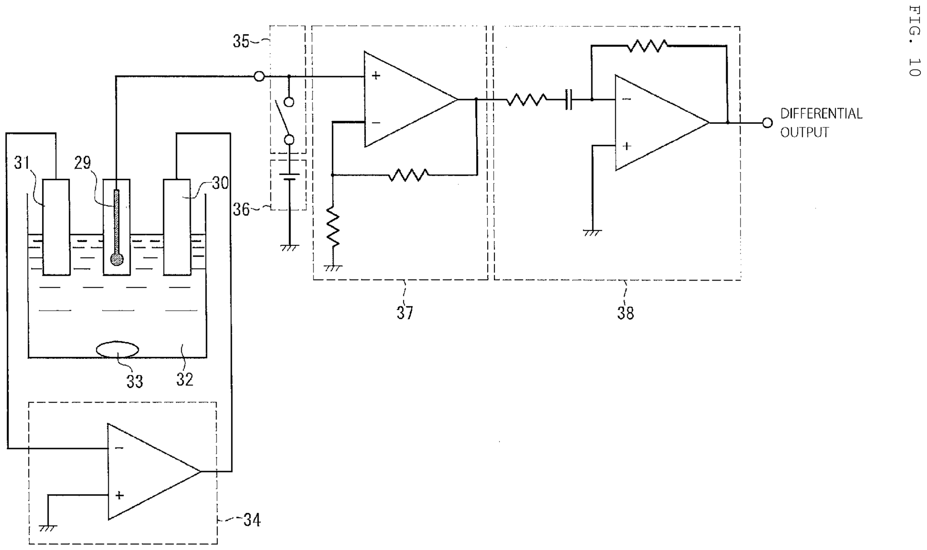

A working electrode 29 (see FIG. 10) and an enzyme-modified electrode including the electrode were each produced in the same manner as in Example 1.

[Counter Electrode]

A platinum electrode was used as a counter electrode 30.

[Reference Electrode (Standard Electrode)]

A silver/silver chloride electrode was used as a reference electrode 31.

[Method of measuring Concentration of Substrate]

FIG. 10 shows another aspect of the measuring system for the concentration of the subject substance (substrate).

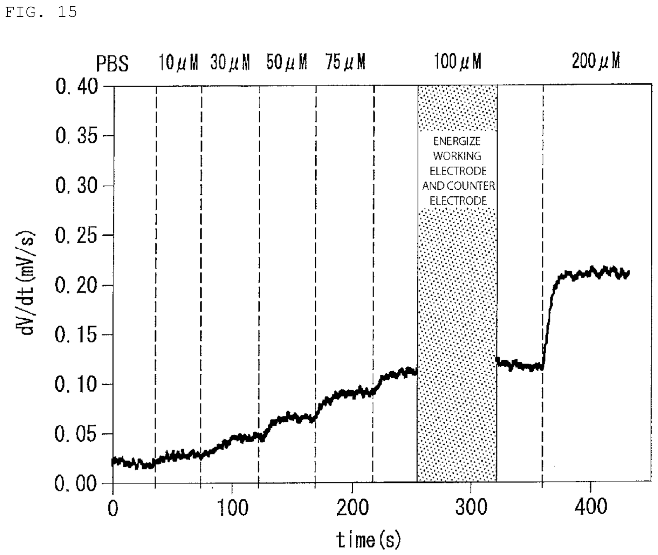

As shown in FIG. 10, there was used a circuit 34 controlling the potential of the counter electrode 30 so that the reference electrode 31 had a ground potential. The enzyme-modified electrode in which the enzyme and the redox substance were immobilized onto the working electrode 29 was connected to a switch 35, which applied a predetermined voltage to control energization between the working electrode 29 and the counter electrode 30, and the non-inverting input terminal of a non-inverting amplifier circuit 37. The enzyme-modified electrode, the counter electrode 30, and the reference electrode 31 were immersed in 10 mL of a PBS solution (subject solution). The PBS solution was stirred with a stirring bar 33 at 500 rpm. After that, first of all, the switch 35 was closed for 100 seconds so that the working electrode 29 and the counter electrode 30 were energized. The potential of the working electrode 29 with respect to the reference electrode 31 at this time was set to 0.015 V. Next, the switch 35 was opened so that the working electrode 29 and the counter electrode 30 were not energized, followed by the start of the measurement of the time differential value dV/dt of the potential of the working electrode 29 output by a differentiating circuit 38. 40 seconds after that, 1 $\mu$L of a 100 mM D-glucose solution was added to the PBS solution, which was being stirred, to adjust the concentration of glucose in the subject

solution to 10 μM. After that, the glucose concentration was continuously changed to 30 μM, 50 μM, 75 μM, 100 μM, and 200 μM. As the potential of the working electrode reached 0.08 V during the measurement of the PBS solution having a glucose concentration of 100 μM, the measurement of the time differential of the working electrode potential with the differentiating circuit 38 was stopped, and the switch 35 was closed for 80 seconds so that the working electrode 29 and the counter electrode 30 were energized. Thus, the potential of the working electrode 29 was returned to 0.015 V. Immediately after the potential of the working electrode 29 had returned to 0.015 V, the switch 35 was opened so that the working electrode 29 and the counter electrode 30 were not energized, followed by the restart of the measurement of the time differential value dV/dt of the potential of the working electrode 29 with the differentiating circuit 38. Thus, there was obtained a graph in which the time differential value dV/dt of the potential of the working electrode 29 for each glucose concentration was continuously measured as shown in FIG. 15. Further, it was able to be recognized that the glucose concentration was able to be measured in real time.

[Determination of Amount of Subject substance]

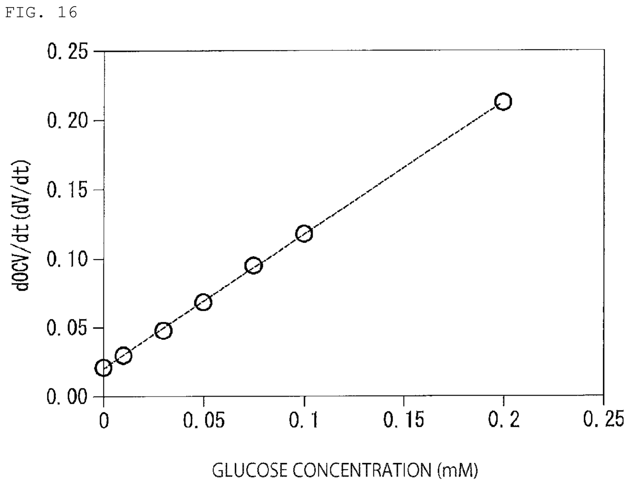

Plots (calibration curve data) in which the glucose concentration and the time differential value dV/dt were in a proportional relationship as shown in FIG. 16 were obtained from the time differential value dV/dt for each glucose concentration shown in FIG. 15. The plots were consistent with the linear function (i). Then, the linear function (i) was stored in the memory of the above-mentioned external information processing terminal (digital electronic calculator), and a program for calculating the absolute value of the concentration of the substrate through use of the linear function (i), the program being similarly stored in the memory, was executed. Thus, the glucose concentration corresponding to the time differential value dV/dt was able to be output.

(Example 4)

[Working Electrode]

The working electrode 21 (see FIG. 8) and an enzyme-modified electrode including the electrode were each produced in the same manner as in Example 1.

[Counter Electrode (Standard Electrode)]

A silver/silver chloride electrode was used as the counter electrode 22.

[Measurement of Electrode Potential and Measurement of Time Period for which Continuous Measurement can be performed]

Such a measuring system as shown in FIG. 8 was used, and the enzyme-modified electrode in which the enzyme and the redox substance were immobilized onto the working electrode 21 was connected to the non-inverting input terminal of the non-inverting amplifier circuit 27. The counter electrode 22 was connected so as to have a ground potential. The enzyme-modified electrode and the counter electrode 22 were immersed in 10 mL of a PBS solution (subject solution), and the subject solution was stirred with the stirring bar 24 at 500 rpm. First of all, the potential of the working electrode 21 output by the non-inverting amplifier circuit 27 was measured. As a result, the potential was 0.015 V. Next, 10 μL of a 100 mM D-glucose solution was added to the PBS solution, which was being stirred, to adjust the concentration of glucose in the subject solution to 10 μM. After that, the time period for which the time differential value dV/dt of the potential for which the linear function (i) was valid was able to be continuously measured was measured. As a result, the time period was 70 seconds.

(Example 5)

[Working Electrode]

The working electrode 21 (see FIG. 8) was produced in the same manner as in Example 4.
An enzyme-modified electrode was produced in the same manner as in Example 1 except that 5 μl of a mixed solution obtained by mixing a 0.2 wt% aqueous dispersion of carbon nanotubes (manufactured by TUBALL) and an aqueous solution (Prussian blue content: 1 mass%), which had been obtained by adding Prussian blue (redox substance, manufactured by Sigma-Aldrich Co. LLC) to an aqueous solution of ascorbic acid (reducing agent, manufactured by Tokyo Chemical Industry Co., Ltd.) adjusted to 100 mM, at a volume ratio of 10:1 was dropped onto the circular section 21a having a diameter of 3 mm out of the working electrode 21 (see FIG. 7). The molar ratio (reducing agent/redox substance) of ascorbic acid to Prussian blue was set to 8.6.

[Counter Electrode (Standard Electrode)]

A silver/silver chloride electrode was used as the counter electrode 22.

[Measurement of Electrode Potential and Measurement of Time Period for which Continuous Measurement can be performed]

Such a measuring system as shown in FIG. 8 was used, and the enzyme-modified electrode in which the enzyme and the redox substance were immobilized onto the working electrode 21 was connected to the non-inverting input terminal of the non-inverting amplifier circuit 27. The counter electrode 22 was connected so as to have a ground potential. The enzyme-modified electrode and the counter electrode 22 were immersed in 10 mL of a PBS solution (subject solution), and the subject solution was stirred with the stirring bar 24 at 500 rpm. First of all, the potential of the working electrode 21 output by the non-inverting amplifier circuit 27 was measured. As a result, the potential was -0.05 V. Next, 10 μL of a 100 mM D-glucose solution was added to the PBS solution, which was being stirred, to adjust the concentration of glucose in the subject solution to 100 μM. After that, the time period for which the time differential value dV/dt of the potential for which the linear function (i) was valid was able to be continuously measured was measured. As a result, the time period was 200 seconds.

(Example 6)

[Working Electrode]

The working electrode 21 (see FIG. 8) was produced in the same manner as in Example 4.
An enzyme-modified electrode including the working electrode 21 was produced in the same manner as in Example 4 except that 5 μl of a mixed solution obtained by mixing a 0.2 wt% aqueous dispersion of carbon nanotubes (manufactured by TUBALL) and an aqueous solution (Prussian blue content: 1 mass%), which had been obtained by adding Prussian blue (redox substance, manufactured by Sigma-Aldrich Co. LLC) to an aqueous solution of 3-mercapto-1,2-propanediol (reducing agent, manufactured by FUJIFILM Wako Pure Chemical Corporation) adjusted to 50 mM, at a volume ratio of 10:1 was dropped onto the circular section 21a having a diameter of 3 mm out of the working electrode 21. The molar ratio (reducing agent/redox substance) of 3-mercapto-1,2-propanediol to Prussian blue was set to 5.0.

[Counter Electrode (Standard Electrode)]

A silver/silver chloride electrode was used as the counter electrode 22.

[Measurement of Electrode Potential and Measurement of Time Period for which Continuous Measurement can be performed]

Such a measuring system as shown in FIG. 8 was used, and the enzyme-modified electrode in which the enzyme and the redox substance were immobilized onto the working electrode 21 was connected to the non-inverting input terminal of the non-inverting amplifier circuit 27. The counter electrode 22 was connected so as to have a ground potential. The enzyme-modified electrode and the counter electrode 22 were immersed in 10 mL of a PBS solution (subject solution), and the subject solution was stirred with the stirring bar 24 at 500 rpm. First of all, the potential of the working electrode 21 output by the circuit 27 was measured. As a result, the potential was -0.04 V. Next, 10 μL of a 100 mM D-glucose solution was added to the PBS solution, which was being stirred, to adjust the concentration of glucose in the subject solution to 100 μM. After that, the time period for which the time differential value dV/dt of the potential for which the linear function (i) was valid was able to be continuously measured was measured. As a result, the time period was 150 seconds.

(Example 7)

[Working Electrode]

The working electrode 21 (see FIG. 8) was produced in the same manner as in Example 4.
An enzyme-modified electrode including the working electrode 21 was produced in the same manner as in Example 4 except that 5 μl of a mixed solution obtained by mixing a 0.2 wt% aqueous dispersion of carbon nanotubes (manufactured by TUBALL) and an aqueous solution (Prussian blue content: 1 mass%), which had been obtained by adding Prussian blue (redox substance, manufactured by Sigma-Aldrich Co. LLC) to an aqueous solution of thiourea (reducing agent, manufactured by FUJIFILM Wako Pure Chemical Corporation) adjusted to 10 mM, at a volume ratio of 10:1 was dropped onto the circular section 21a having a diameter of 3 mm out of the working electrode 21. The molar ratio (reducing agent/redox substance) of thiourea to Prussian blue was set to 3.0.

[Counter Electrode (Standard Electrode)]

A silver/silver chloride electrode was used as the counter electrode 22.

[Measurement of Electrode Potential and Measurement of Time Period for which Continuous Measurement can be performed]

Such a measuring system as shown in FIG. 8 was used, and the enzyme-modified electrode in which the enzyme and the redox substance were immobilized onto the working electrode 21 was connected to the non-inverting input terminal of the non-inverting amplifier circuit 27. The counter electrode 22 was connected so as to have a ground potential. The enzyme-modified electrode and the counter electrode 22 were immersed in 10 mL of a PBS solution (subject solution), and the subject solution was stirred with the stirring bar 24 at 500 rpm. First of all, the potential of the working electrode 21 output by the circuit 27 was measured. As a result, the potential was -0.02 V. Next, 10 μL of a 100 mM D-glucose solution was added to the PBS solution, which was being stirred, to adjust the concentration of glucose in the subject solution to 100 μM. After that, the time period for which the time differential value dV/dt of the potential for which the linear function (i) was valid was able to be continuously measured was measured. As a result, the time period was 100 seconds.

Table 1

| Table 1 | Reducing agent in redox layer | Molar ratio (reducing agent/redox substance) | Electrode potential before glucose addition (V) | Time period for which continuous measurement can be performed (seconds) |
|---|---|---|---|---|
| Example 1 | - | - | 0.015 | 70 |
| Example 5 | L-Ascorbic acid | 8.6 | -0.05 | 200 |
| Example 6 | Mercaptopropanediol | 5.0 | -0.04 | 150 |
| Example 7 | Thiourea | 3.0 | -0.02 | 100 |

As can be seen from comparison between Example 4 and each of Examples 5 to 7, when the reducing agent is previously incorporated into the redox layer, the time period for which the time differential value dV/dt of the potential for which the linear function (i) is valid can be continuously measured can be lengthened.

Industrial Applicability

The measurement of a substrate concentration with the enzyme sensor of the present disclosure is the measurement of the substrate concentration through utilization of the potential of its working electrode, and hence there is no need to flow an electric current through the working electrode or a solution containing a subject substance. Accordingly, the sensor is useful in, for example, the case where the substrate concentration is low (e.g., several tens of μM) or the case where the concentrations of a plurality of substrates are simultaneously measured.

Explanation of References

1 enzyme sensor
2, 21, 29 working electrode
3, 17, 22, 30 counter electrode
4 enzyme-immobilized layer
5 redox substance layer
6, 19, 23, 32 subject solution
7 base material

8 sensing part
9 dV/dt output mechanism
101 measuring section
10a, 10b circuit
11, 31 reference electrode
12 display section
13 computing unit
14 central processing unit
15 storage section
15a main storage section
15b auxiliary storage section
16 signal processing mechanism
20, 24, 33 stirring bar
25, 35 working electrode-counter electrode energization switch
26, 36 power supply
27, 37 non-inverting amplifier circuit
28, 38 differentiating circuit
34 circuit

**Claims**

1. An enzyme sensor for measuring a concentration of a subject substance in a solution containing the subject substance, comprising:

   a working electrode and a standard electrode, each of which may be brought into contact with the solution;
   an enzyme and a redox substance capable of being reversibly oxidized and reduced, the enzyme and the redox substance being each immobilized onto the working electrode; and
   a dV/dt output mechanism capable of outputting, with time, a time differential value dV/dt of a potential difference between the standard electrode and the working electrode, which is caused by changes in concentrations of an oxidized form and a reduced form of the redox substance resulting from a reaction between the subject substance and the enzyme,
   wherein the potential difference shifts to higher values when the redox substance is oxidized by the reaction between the subject substance and the enzyme, or the potential difference shifts to lower values when the redox substance is reduced by the reaction.

2. The enzyme sensor according to claim 1, wherein the redox substance is oxidized or reduced by a product produced by the reaction between the subject substance and the enzyme.

3. The enzyme sensor according to claim 1 or 2, wherein the enzyme is an enzyme capable of exchanging an electron through the reaction.

4. The enzyme sensor according to any one of claims 1 to 3, comprising a mechanism for calculating, with time, an absolute value of the concentration of the subject substance corresponding to the time differential value dV/dt through the following linear function (i) for calculating the absolute value of the concentration of the subject substance from the time differential value dV/dt:

$$c_M = A\frac{dV}{dt} \qquad\qquad (i)$$

in the linear function (i), V represents the potential difference between the standard electrode and the working electrode, t represents time, $c_M$ represents the concentration of the subject substance, and A represents a proportionality constant.

5. The enzyme sensor according to any one of claims 1 to 4, wherein an enzyme-modified electrode in which the enzyme and the redox substance are immobilized onto the working electrode,

further contains a reducing agent when the redox substance is oxidized by the reaction between the subject substance and the enzyme, or
further contains an oxidizing agent when the redox substance is reduced by the reaction between the subject substance and the enzyme.

6. The enzyme sensor according to any one of claims 1 to 5, wherein the potential difference shifts to higher values through oxidation of the redox substance by the reaction between the subject substance and the enzyme.

7. The enzyme sensor according to any one of claims 1 to 6,

wherein an electrode system of the enzyme sensor is a two-electrode system formed of the working electrode and a counter electrode, or a three-electrode system formed of the working electrode, the counter electrode, and a reference electrode, and
wherein the standard electrode is the counter electrode for forming the two-electrode system or the reference electrode for forming the three-electrode system.

8. The enzyme sensor according to any one of claims 1 to 7, wherein the redox substance forms a redox layer disposed in contact with the working electrode, and the enzyme forms an enzyme-immobilized layer disposed in contact with the redox layer.

9. The enzyme sensor according to any one of claims 1 to 8, wherein the redox layer contains a conductive carbon material.

10. The enzyme sensor according to claim 9, wherein the conductive carbon material contains a carbon nanotube.

11. The enzyme sensor according to claim 7, further comprising, in each of the two-electrode system and the three-electrode system, a circuit for applying a predetermined voltage between the counter electrode and the working electrode to enable flow of an electric current between the working electrode and the counter electrode.

12. The enzyme sensor according to claim 4, further comprising a display section for displaying the calculated absolute value of the concentration of the subject substance.

13. A method for measurement of a subject substance with the enzyme sensor according to any one of claims 1 to 12, comprising:

bringing a solution containing the subject substance into contact with both the working electrode and the standard electrode;
obtaining, with time, a time differential value dV/dt of a potential difference between the standard electrode and the working electrode under a state in which the working electrode and the standard electrode are electrically connected to each other, and a voltage is free from being applied between the working electrode and the standard electrode; and
calculating a concentration of the subject substance through use of the time differential value dV/dt thus obtained and the following linear function (i):

$$c_M = A \frac{dV}{dt} \qquad (i)$$

in the linear function (i), V represents the potential difference between the standard electrode and the working electrode, t represents time, $C_M$ represents the concentration of the subject substance, and A represents a proportionality constant.

14. A method for measurement of a subject substance with the enzyme sensor according to claim 11, comprising:

when the electrode system of the enzyme sensor is the two-electrode system, bringing a solution containing the subject substance into contact with the working electrode and the counter electrode, or when the electrode system is the three-electrode system, bringing the solution containing the subject substance into contact with the working electrode, the counter electrode, and the reference electrode;

at a time of reaching of a potential of the working electrode to a predetermined potential, which has previously been set, wherein the working electrode and the counter electrode are electrically connected when the electrode system of the enzyme sensor is the two-electrode system, or the working electrode, the counter electrode and the reference electrode are electrically connected when the electrode system of the enzyme sensor is the three-electrode system, applying a predetermined voltage between the counter electrode and the working electrode in each of the two-electrode system and the three-electrode system to flow an electric current between the working electrode and the counter electrode, to return the potential of the working electrode to a value before a start of the measurement;
obtaining, with time, a time differential value dV/dt of a potential difference between the standard electrode and the working electrode; and
calculating a concentration of the subject substance through use of the time differential value dV/dt thus obtained and the following linear function (i):

$$c_M = A\frac{dV}{dt} \qquad (i)$$

in the linear function (i), V represents the potential difference between the standard electrode and the working electrode, t represents time, $C_M$ represents the concentration of the subject substance, and A represents a proportionality constant.

15. A measuring system for measuring a concentration of a subject substance in a solution containing the subject substance, the measuring system comprising:

an enzyme sensor;
a signal processing mechanism; and
a display section,
wherein the enzyme sensor includes:

a working electrode and a standard electrode, each of which may be brought into contact with the solution;
an enzyme and a redox substance capable of being reversibly oxidized and reduced, the enzyme and the redox substance being each immobilized onto the working electrode; and
a measuring section capable of measuring, with time, a potential difference between the standard electrode and the working electrode, which is caused by changes in concentrations of an oxidized form and a reduced form of the redox substance resulting from a reaction between the subject substance and the enzyme,

wherein the potential difference shifts to higher values when the redox substance is oxidized by the reaction between the subject substance and the enzyme, or the potential difference shifts to lower values when the redox substance is reduced by the reaction,
wherein the signal processing mechanism calculates the concentration of the subject substance from the potential difference through use of the following linear function (i), and
wherein the display section is capable of displaying the concentration of the subject substance calculated by the signal processing mechanism:

$$c_M = A\frac{dV}{dt} \qquad (i)$$

in the linear function (i), V represents the potential difference between the standard electrode and the working electrode, t represents time, $C_M$ represents the concentration of the subject substance, and A represents a proportionality constant.

FIG. 1

FIG. 2

FIG. 3

FIG. 4

FIG. 5

FIG. 6

FIG. 7

FIG. 8

DIFFERENTIAL
OUTPUT

EP 4 715 378 A1

FIG. 9

FIG. 10

EP 4 715 378 A1

DIFFERENTIAL
OUTPUT

FIG. 11

FIG. 12

FIG. 13

FIG. 14

FIG. 15

FIG. 16

## INTERNATIONAL SEARCH REPORT

| International application No. |
| --- |
| **PCT/JP2024/018075** |

### A. CLASSIFICATION OF SUBJECT MATTER

*G01N 27/327*(2006.01)i; *G01N 27/416*(2006.01)i
FI: G01N27/327 353R; G01N27/416 336B

According to International Patent Classification (IPC) or to both national classification and IPC

### B. FIELDS SEARCHED

Minimum documentation searched (classification system followed by classification symbols)

G01N27/327; G01N27/416

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); Scopus

### C. DOCUMENTS CONSIDERED TO BE RELEVANT

| Category* | Citation of document, with indication, where appropriate, of the relevant passages | Relevant to claim No. |
| --- | --- | --- |
| X | JP 62-240849 A (OMRON CORPORATION) 21 October 1987 (1987-10-21) p. 1, left column, 5th paragraph to right column, 1st paragraph, p. 3, upper left column, 1st paragraph, upper right column, 4th paragraph to lower left column, 3rd paragraph, p. 4, upper left column, 1st paragraph to upper right column, 3rd paragraph, lower left column, 2nd paragraph, lower left column, 5th paragraph to lower right column, 2nd paragraph, fig. 1-4, formula 2 | 1-3, 5-7, 11, 13-15 |
| Y | | 4, 8-10, 12 |
| Y | JP 2015-79004 A (ARKRAY, INC.) 23 April 2015 (2015-04-23) paragraphs [0009], [0010], [0064]-[0069], [0088], fig. 6, 8 | 4, 8-10, 12 |
| Y | JP 2010-54378 A (TANITA CORPORATION) 11 March 2010 (2010-03-11) paragraphs [0007], [0071], fig. 3 | 8 |
| Y | JP 2021-113821 A (THE NATIONAL INSTITUTE OF ADVANCED INDUSTRIAL SCIENCE AND TECHNOLOGY) 05 August 2021 (2021-08-05) paragraphs [0021], [0022], [0036] | 9-10 |

☑ Further documents are listed in the continuation of Box C.    ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **19 June 2024** | **02 July 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

| International application No. |
|---|
| **PCT/JP2024/018075** |

**C.    DOCUMENTS CONSIDERED TO BE RELEVANT**

| Category* | Citation of document, with indication, where appropriate, of the relevant passages | Relevant to claim No. |
|---|---|---|
| A | JP 2019-132705 A (ASAHI KASEI KABUSHIKI KAISHA) 08 August 2019 (2019-08-08) paragraph [0099] | 4, 12 |
| A | JP 2008-128803 A (HITACHI, LTD.) 05 June 2008 (2008-06-05) entire text, all drawings | 1-15 |
| A | JP 2019-203893 A (ARKRAY, INC.) 28 November 2019 (2019-11-28) entire text, all drawings | 1-15 |
| A | JP 62-235557 A (OMRON CORPORATION) 15 October 1987 (1987-10-15) entire text, all drawings | 1-15 |
| A | SONG, Y. et al. Design and preparation of open circuit potential biosensor for in vitro and in vivo glucose monitoring. Anal Bioanal Chem, 2017, vol. 409, pp. 161-168, doi.org/10.1007/s00216-016-9982-1 entire text, all drawings | 1-15 |

Form PCT/ISA/210 (second sheet) (July 2022)

EP 4 715 378 A1

INTERNATIONAL SEARCH REPORT
Information on patent family members

International application No.

**PCT/JP2024/018075**

| Patent document cited in search report | | | Publication date (day/month/year) | Patent family member(s) | Publication date (day/month/year) |
|---|---|---|---|---|---|
| JP | 62-240849 | A | 21 October 1987 | (Family: none) | |
| JP | 2015-79004 | A | 23 April 2015 | US 2011/0259741 A1 paragraphs [0013], [0014], [0082]-[0087], [0106], fig. 6, 8 JP 2011-242385 A | |
| JP | 2010-54378 | A | 11 March 2010 | (Family: none) | |
| JP | 2021-113821 | A | 05 August 2021 | US 2019/0194714 A1 paragraphs [0032]-[0038], [0069] WO 2018/043050 A1 EP 3505626 A1 CN 109642220 A JP 2018-33325 A | |
| JP | 2019-132705 | A | 08 August 2019 | (Family: none) | |
| JP | 2008-128803 | A | 05 June 2008 | US 2008/0116070 A1 entire text, all drawings US 2012/0261259 A1 | |
| JP | 2019-203893 | A | 28 November 2019 | US 2019/0360018 A1 entire text, all drawings EP 3578665 A1 CN 110514704 A | |
| JP | 62-235557 | A | 15 October 1987 | (Family: none) | |

Form PCT/ISA/210 (patent family annex) (July 2022)

38

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6488244 A
- JP 2019158650 A

- JP 2023553889 B